# FASCICULE DE BREVET EUROPEEN

(11) **EP 2 213 334 B1**
(45) Date de publication et mention de la délivrance du brevet: **30.09.2015**
(21) Numéro de dépôt: 10151680.5
(22) Date de dépôt: 26.01.2010
(51) Int. Cl.: A61Q 5/12, A61K 8/58, A61Q 5/06, A61K 8/73

(54) **Composition cosmetique comprenant un alkoxysilane particulier et une gomme microbienne et utilisations en coiffage**
Kosmetische Zusammensetzung, die eine spezifische Alkoxysilan und eine antibakterielle Gum enthält; Verwendung dieser Zusammensetzung für Haarstyling
Cosmetic composition comprising a specific alkoxysilane and an antibacterial gum; use of said composition for styling hair.

(30) Priorité: 30.01.2009 FR 0950604
(43) Date de publication de la demande: 04.08.2010
(73) Titulaire: L'Oréal, 75008 Paris (FR)
(72) Inventeur: Benabdillah, Katarina, 30140, Saint Jean Du Pin (FR); Lerda, Patrice, 92600, Asnieres (FR)
(74) Mandataire: Casalonga, Axel

(56) Documents cités:
- EP-A- 1 532 967
- EP-A- 1 767 189
- EP-A- 1 935 398
- FR-A- 2 783 165
- FR-A- 2 798 063
- FR-A- 2 910 276

## Description

La présente invention se rapporte à des compositions cosmétiques comprenant un ou plusieurs alcoxysilane(s) à fonction(s) basique(s) et une ou plusieurs gomme(s) microbienne(s), et à leur utilisation pour la mise en forme des fibres kératiniques.

Les compositions de traitement capillaire contenant des alcoxysilanes sont connues de l'art antérieur.

La demande de brevet EP-159 628 A2 décrit une composition de permanente, et de fortification des cheveux contenant un alkyl trialcoxy silane.

La demande de brevet FR 2 783 164 décrit l'utilisation de composés organiques du silicium choisis parmi les silanes comprenant un atome de silicium et les siloxanes comprenant deux ou trois atomes de silicium, ces composés étant hydrosolubles, pour le maintien de la coiffure où la mise en forme des cheveux et la demande de brevet FR 2 891 142 décrit l'utilisation d'un ou plusieurs composés organiques du silicium, de préférence non hydrosolubles, et solubles dans les milieux alcooliques ou hydroalcooliques.

Ces compositions permettent d'obtenir des effets coiffants notamment sur cheveux fins et frisés. Néanmoins, ces effets n'étant pas suffisamment intenses et durables, il est généralement nécessaire de réappliquer les produits après plusieurs jours.

On a également observé qu'après plusieurs applications c'est-à-dire plusieurs superpositions des compositions contenant des alcoxysilanes à fonctions solubilisantes, les cheveux peuvent devenir secs, rêches, avoir un toucher de cheveux sensibilisés, abîmés.

De manière surprenante, la Demanderesse a découvert que des compositions cosmétiques comprenant un ou plusieurs alcoxysilane(s) à fonction(s) basique(s) et une ou plusieurs gomme(s) microbienne(s) permettent de résoudre ces problèmes.

Après application des compositions selon l'invention sur les cheveux on obtient un effet coiffant satisfaisant et durable. La coiffure présente du corps et du volume. Les cheveux présentent une grande facilité de démêlage, une grande souplesse ainsi qu'un toucher doux et lisse. Ces effets résistent à plusieurs shampooings.

Ces effets sont particulièrement visibles sur les cheveux fins ou sur cheveux abîmés voire très abîmés par exemple sur cheveux décolorés.

Sur cheveux frisés, secs, les compositions selon l'invention permettent aussi l'obtention d'un contrôle de la mise en forme, en particulier un contrôle du dessin des boucles.

L'invention a donc pour objet une composition cosmétique non détergente comprenant un ou plusieurs alcoxysilane(s) présentant une ou plusieurs fonction(s) basique(s) et une ou plusieurs gomme(s) microbienne(s).

L'invention concerne encore l'utilisation pour le traitement des matières kératiniques, notamment des cheveux, et en particulier des cheveux abîmés, frisés, secs ou fins, d'une composition cosmétique non détergente comprenant un ou plusieurs alcoxysilanes à fonction(s) basique(s) et une ou plusieurs gomme(s) microbienne(s).

L'invention se rapporte enfin à un procédé de mise en forme des cheveux comprenant l'application sur les cheveux de ladite composition.

Un autre objet de l'invention concerne l'utilisation de cette composition pour éviter la dégradation du toucher des cheveux générée par les applications répétées de compositions de mise en forme des cheveux contenant un ou plusieurs alcoxysilanes à fonction(s) basique(s).

D'autres caractéristiques, propriétés et avantages de la présente invention apparaîtront plus clairement à la lecture de la description et des exemples qui suivent.

Par « composition non détergente » au sens de la présente demande, on entend que la composition ne contient pas plus de 4% en poids d'agents tensio-actifs par rapport au poids total de la composition.

Les alcoxysilanes à fonction(s) basique(s) présents dans la composition selon l'invention sont choisis parmi les organosilanes comprenant un, deux ou trois atomes de silicium, de préférence un ou deux atomes de silicium. Ils doivent en outre comporter une ou plusieurs fonctions chimiques basiques. La fonction chimique basique peut correspondre à toute fonction conférant un caractère basique au composé de silicium et est, de préférence, une fonction amine telle qu'une fonction amine primaire, secondaire ou tertiaire. La fonction chimique basique des composés du silicium selon l'invention, peut comporter éventuellement d'autres fonctions, telles que, par exemple, une autre fonction amine, une fonction acide ou une fonction halogène.

Les alcoxysilanes à fonction(s) basique(s) présents dans la composition selon l'invention comportent en outre deux ou plusieurs groupes hydrolysables ou hydroxyles par molécule. Les groupes hydrolysables sont de préférence des groupes alcoxy, aryloxy ou halogène. Ils peuvent également, éventuellement, comporter d'autres fonctions chimiques telles que des fonctions acides.

Selon un mode de réalisation particulier, le ou les alcoxysilane(s) à fonction(s) basique(s) présent(s) dans la composition selon l'invention sont choisis parmi les composés de formule (1) : 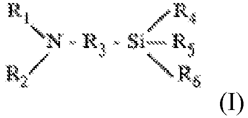 dans laquelle :
R₄ représente un halogène, un groupe OR' ou R'₁ ;
R₅ représente un halogène, un groupe OR" ou R'₂ ;
R₆ représente un halogène, un groupe OR"' ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires tels que des groupes acides ou amines, R₁, R₂, R', R" et R"' pouvant en outre désigner l'hydrogène, et deux au moins des groupes R₄, R₅ et R₆ étant différents des groupes R'₁, R'₂ et R'₃.

De préférence, les groupes R₁, R₂, R', R'₁, R'₂, R'₃, R" et R"' sont choisis parmi les radicaux alkyle de C₁-C₁₂, aryle de C₅ à C₁₄, alkyl(C₁-C₈)aryle de C₅ à C₁₄, et aryl(C₅-C₁₄)alkyle de C₁ à C₈.

De préférence, le groupe R₃ est choisi parmi les radicaux alkylène en C₁-C₁₂, éventuellement substitué par un groupement amino, arylène en C₅-C₁₄, alkylène(C₁-C₈)arylène en C₅-C₁₄, et arylène(C₅-C₁₄)alkylène en C₁-C₈.

Selon un mode de réalisation particulier, le ou les alcoxysilane(s) à fonction(s) basique(s) répondant aux formules (I) sont de préférence le 3-aminopropyltriéthoxysilane, le 3-aminopropylméthyldiéthoxysilane, le N-(2-aminoéthyl)-3-aminopropylltriéthoxysilane, et le 3-(2-aminoéthylamino)propyl-méthyldiéthoxysilane.

Selon un autre mode de réalisation, le ou les alcoxysilane(s) à fonction(s) basique(s) utilisé(s) selon l'invention sont choisis parmi les composés de formule (II) :

(R₂₁O)ₓ(R₂₂)_{y}Si-(B)ₚ-[NR₂₃-(B')ₚ,]_{q}-[NR'₂₃-(B")_{p"}]_{q'}-(R'₂₂)_{y'}(OR'₂₁)ₓ, (II)

dans laquelle :
R₂₁, R₂₂, R'₂₁ et R'₂₂ représentent chacun indépendamment, l'un de l'autre, une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x est un entier variant de 1 à 3, y = 3-x, x' est un entier variant de 1 à 3, y' = 3-x', p = 0 ou 1, p' = 0 ou 1, p" = 0 ou 1, q = 0 ou 1, q' = 0 ou 1, étant entendu que au moins q ou q' est différent de zéro,
B, B' et B" représentent chacun indépendamment un radical divalent alkylène linéaire ou ramifié en C₁-C₂₀,
R₂₃ et R'₂₃ représentent chacun indépendamment un atome d'hydrogène ou une chaîne hydrocarbonée saturée ou insaturée, linéaire ou ramifiée, contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₃-C₂₀, amine, amide, carboxyle, alcoxysilane, hydroxyle, carbonyle ou acyle.

Comme expliqué précédemment, R₂₁, R₂₂, R'₂₁ et R'₂₂ représentent chacun indépendamment une chaîne hydrocarbonée. Par chaîne hydrocarbonée, on entend de préférence une chaîne comportant de 1 à 30 et de préférence 1 à 10 atomes de carbone

De même, R₂₃ et R'₂₃ peuvent représenter une chaîne hydrocarbonée. Dans ce cas, on entend de préférence une chaîne comportant de 1 à 30 et de préférence 1 à 10 atomes de carbone.

De préférence, le cycle aromatique comporte de 6 à 30 atomes de carbone Encore plus préférentiellement, il désigne un radical phényle éventuellement substitué

De préférence, R₂₁ = R'_{21 ;} R₂₂ = R'_{22 ;} x = x' ; y = y' ; p = p' ; B = B' ; q = 1 et q' = 0.

Le ou les alcoxysilane(s) à fonction(s) basique(s) de formule (II) peuvent également présenter les caractéristiques suivantes, prises seules ou en combinaison :
- R₂₁, R₂₂, R'₂₁ et R'₂₂, identiques ou différents, représentent un alkyle en C₁-C₄,
- p=p'=1 ;
- B et B', identiques ou différents représentent un alkylène linéaire en C₁-C₄ et
- R₂₃ est l'hydrogène.

Par exemple, le ou les alcoxysilane(s) à fonction(s) basique(s) peuvent comprendre un substituant comprenant une fonction amine secondaire, comme la bis[3-(triéthoxysilyl)propyl]amine de formule (CH₃CH₂O)₃-Si(CH₂)₃NH(CH₂)₃Si(OCH₂CH₃)₃ proposé par la société Fluorochem, la bis [triméthoxysilylpropyl]amine de formule (CH₃O)₃-Si(CH₂)₃NH(CH₂)₃Si(OCH₃)₃ proposé par la société Gelest, la bis[méthyldiéthoxysilylpropyl]amine de formule (CH₃CH₂O)₂CH₃Si(CH₂)₃NH(CH₂)₃SiCH₃(OCH₂CH₃)₂ proposé par la société Gelest et la bis[3-triméthoxysilylpropyl]éthylènediamine de formule (CH₃O)₃Si(CH₂)₃NH(CH)₂NH(CH₂)₃Si(OCH₃)₃ proposé par la société Gelest. Parmi ces composés, on préfère la bis[3-(triéthoxysilyl)propyl] amine et la bis[méthyldiéthoxysilylpropyl] amine.

Selon un autre mode de réalisation de l'invention, le ou les alcoxysilane(s) à fonction(s) basique(s) sont choisis parmi les composés de formule (III) : 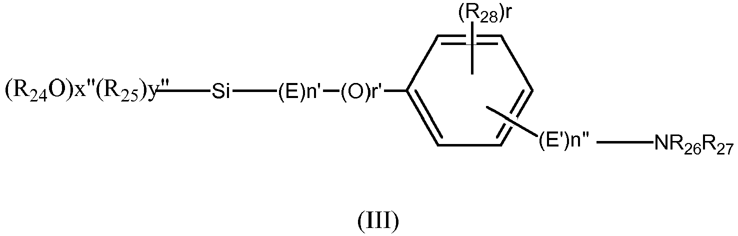 dans laquelle :
R₂₄ et R₂₅ représentent chacun indépendamment, l'un de l'autre, une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x" = 2 ou 3 ;
y" = 3-x" ;
n' = 0 ou 1 ;
n"= 0 ou 1 ;
E et E' représentent chacun indépendamment un radical divalent alkylène linéaire ou ramifié en C₁-C₂₀,
R₂₆ et R₂₇ représentent chacun indépendamment l'hydrogène ou une chaîne hydrocarbonée saturée ou insaturée, linéaire ou ramifiée, contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₁-C₂₀, amine, amide, carboxyle, alcoxysilane, hydroxyle, carbonyle ou acyle,
r est un entier variant de 0 à 4,
r' = 0 ou 1,
le ou les groupes R₂₈ représentent chacun indépendamment l'hydrogène ou une chaîne hydrocarbonée saturée ou insaturée, linéaire ou ramifiée, de préférence en C₁-C₁₀, contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₁-C₂₀, amine, amide, carboxyle, alcoxysilane, hydroxyle, carbonyle ou acyle.

Comme expliqué précédemment, R₂₄ et R₂₅ représentent chacun indépendamment une chaîne hydrocarbonée. Par chaîne hydrocarbonée, on entend de préférence une chaîne comportant de 1 à 30 et de préférence 1 à 10 atomes de carbone.

De même, R₂₆ et R₂₇ peuvent représenter une chaîne hydrocarbonée. Dans ce cas, on entend de préférence une chaîne comportant de 1 à 30 et de préférence 1 à 10 atomes de carbone.

De préférence, le cycle aromatique comporte de 6 à 30 atomes de carbone. Encore plus préférentiellement, il désigne un radical phényle éventuellement substitué.

Le ou les alcoxysilane(s) à fonction(s) basique(s) de formule (III) peuvent présenter les caractéristiques suivantes, prises seules ou en combinaison :
- R₂₄ est un alkyle en en C₁-C₄,
- x" = 3, n'= n"=1 ; r = r'=0,

R₂₆ et R₂₇ représentent indépendamment l'hydrogène ou un groupe choisi parmi les groupes alkyle en C₁-C₄, hydroxyalkyle en C₁-C₄ et aminoalkyle en C₁-C₄.

En particulier, le ou les alcoxysilane(s) à fonction(s) basique(s) de formule (III) peuvent être choisis parmi :
- le 3-(m-aminophénoxy)propyltriméthoxysilane, de formule : 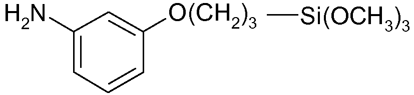
- le p-aminophényltriméthoxysilane, de formule : 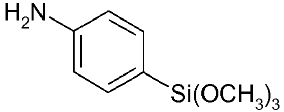
- le N-(2-aminoéthylaminométhyl)phénéthyltriméthoxysilane, de formule :

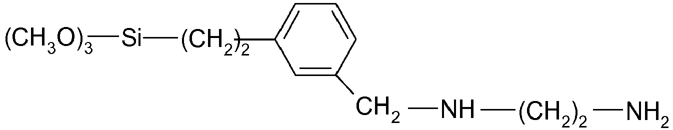

De préférence les alcoxysilanes à fonction(s) basique(s) de l'invention comportent une ou plusieurs fonctions amine primaire ou secondaire.

Encore plus préférentiellement le ou les alcoxysilane(s) à fonction(s) basique(s) utilisables dans les compositions selon la présente invention correspondent à la formule (I) : 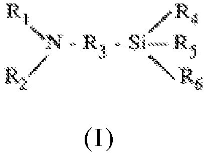 dans laquelle :
R₁ et R₂, indépendamment l'un de l'autre sont choisis parmi l'hydrogène et les groupes éthyle, propyle et aminoéthyle ;
R₃ est choisi parmi les groupes éthyle, propyle et méthylphénéthyle ;
R₄, R₅ et R₆ indépendamment l'un de l'autre sont choisis parmi les groupes méthyle, méthoxy, éthoxy.

De préférence, les alcoxysilanes de formule (I) sont choisis parmi les composés suivants : le 3-aminopropyltriéthoxysilane (APTES), le 3-aminoéthyltriéthoxysilane (AETES), le 3-aminopropylméthyldiéthoxysilane, le N-(2-aminoéthyl)-3-aminopropyltriéthoxysilane et le N-(2-aminoéthylaminométhyl)phénéthyltriméthoxysilane de formule :

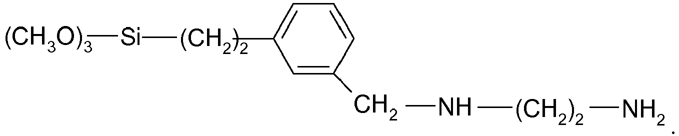

Le ou les alcoxysilane(s) à fonction(s) basique(s) sont généralement utilisés en une quantité allant de 0,1 à 20 %, de préférence entre 1 et 15 % en poids du poids total de la composition.

Au sens de la présente invention, on entend par « gommes microbiennes », des substances synthétisées par fermentation de sucres par des micro-organismes. Les gommes microbiennes peuvent aussi être choisies parmi les gommes de scléroglucane, les gommes de gellane, les gommes de pullulane, les gommes de Curdlar, les gommes de xanthane, les gommes de grifolane, les gommes de lentinane, les gommes de Schizophyllane, les gommes de spirulinane et les gommes de krestine.

Et notamment les gommes de scléroglucane produites par *Sclerotium rolfsii*, les gommes de gellane produites par *Pseudomonas elodea* ou *Sphingomonias,* les gommes de pullulane produites par *Aureobacidium pullulens,* les gommes de Curdlar produites par les alcaligènes de type *Faecalis myxogènes*, les gommes de xanthane produites par de nombreux organismes dont *Leuconostoc mesenteroides* et *Leuconostoc dextrantum*, les gommes de grifolane produites par *Grifola frondara*, les gommes de lentinane produites par *Lentinus edodes,* les gommes de Schizophyllane produites par *Schizophyllum commine*, les gommes de spirulinane produites par *Spirulina sybsyla* et les gommes de krestine produites par *Coriates versicolor.*

On peut aussi, en particulier, citer les gommes de xanthane produites par la bactérie *xanthomonas campestri* et les mutants et variants de celle-ci. Ces gommes de xanthane ont généralement un poids moléculaire compris entre 1 000 000 et 50 000 000.

De préférence, on utilise les gommes de xanthane et de scléroglucane. Encore plus préférentiellement, on utilise les gommes de scléroglucane.

La ou les gommes microbiennes sont en général comprise(s) dans la composition dans des quantités variant de 0,01 à 10 %, de préférence entre 0,1 et 4 %, en poids du poids total de la composition.

De préférence, le rapport pondéral entre la ou les gomme(s) microbienne(s) et le ou les alcoxysilane(s) à fonction(s) basique(s) va de 1/20 à 1/3.

Les compositions selon la présente invention peuvent également comprendre un ou plusieurs agents épaississants additionnels différents des gommes microbiennes et convenant pour les compositions de l'invention comme par exemple les polymères et copolymères carboxyvinyliques, les polymères et copolymères (alkyl)acryliques, les polymères et copolymères (alkyl)acrylamides, les poly(oxyalkylène)glycols, les esters de poly(oxyalkylène)glycols, les dérivés de cellulose, les alginates, les dérivés d'amidon, les gommes naturelles telles que les gommes de guar ou de caroube, les dérivés de chitine et de chitosane, les carraghénanes, les argiles, et leur mélanges.

Les dérivés de cellulose sont par exemple des hydroxyalkylcelluloses et en particulier des hydroxyéthylcelluloses, des hydroxypropylcellulose, ou hydroxypropylméthylcelluloses.

Ces polymères épaississant peuvent le cas échéant comporter une ou plusieurs chaînes grasses. On peut ainsi citer les cétylhydroxyéthylcelluloses.

Les agents épaississants additionnels représentent en général de 0,05 à 15%, de préférence de 0,5 à 10% en poids de la composition.

La composition cosmétique selon l'invention peut comprendre en outre un ou plusieurs solvants organiques, de préférence dans une quantité comprise entre 0,05 et 40%, de manière tout à fait préférée, entre 1 et 20% en poids, par rapport au poids total de la composition.

Ce solvant organique peut être un alcool inférieur en C₂ à C₄, en particulier l'éthanol, un polyol tel que le propylène glycol ou le glycérol ou un éther de polyol.

De manière préférée, le milieu est aqueux ou hydroalcoolique.

Les compositions selon l'invention peuvent également contenir d'autres adjuvants cosmétiquement acceptables, tels que par exemple des agents tensioactifs, des agents co-épaississants, des agents de pénétration, des parfums, des colorants, des plastifiants, des tampons, et divers adjuvants usuels comme des cires, des silicones volatiles ou non, cycliques ou linéaires ou ramifiées, organomodifiées (notamment par des groupements amines) ou non différentes des alcoxysilane(s) à fonction(s) basique(s) de l'invention, des céramides, des pseudocéramides, des huiles végétales, minérales ou de synthèse, les vitamines ou provitamines comme le panthénol, des opacifiants, des agents réducteurs, des émulsionnants, des conservateurs, des charges minérales, des nacres, des paillettes, des filtres solaires, des protéines, des polymères fixants anioniques, non ioniques, cationiques ou amphotères, des agents hydratants, des émollients, des agents adoucissants, des agents anti-mousse, des agents antiperspirants, des agents anti-radicaux libres, des bactéricides, des séquestrants, des antipelliculaires, des anti-oxydants, des agents alcalinisants, des acides, des parfums et tout autre additif classiquement utilisé dans les compositions cosmétiques destinées à être appliquées sur les cheveux.

Les agents tensioactifs utilisables dans la composition selon la présente invention peuvent être des tensioactifs anioniques, non ioniques, amphotères ou cationiques, ou des mélanges de ceux-ci.

Parmi les tensioactifs anioniques utilisables, seuls ou en mélanges, dans le cadre de la présente invention, on peut notamment citer les sels, et en particulier les sels de métaux alcalins tels que les sels de sodium, les sels d'ammonium, les sels d'amines, les sels d'amino-alcools ou les sels de métaux alcalino-terreux, par exemple, de magnésium, des composés suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkyl-arylsulfonates, les α-oléfine-sulfonates, les paraffine-sulfonates ; les alkylsulfosuccinates, les alkyléthersulfosuccinates, les alkylamide-sulfosuccinates ; les alkylsulfoacétates ; les acylsarconisates ; et les acylglutamates, les groupes alkyle et acyle de tous ces composés comportant de 6 à 24 atomes de carbone et le groupe aryle désignant de préférence un groupe phényle ou benzyle.

On peut également utiliser dans le cadre de la présente invention les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Les agents tensioactifs non-ioniques que l'on peut utiliser dans le cadre de la présente invention, sont eux aussi, des composés bien connus en soi (voir notamment à cet égard "Handbook of Surfactants" par M.R. PORTER, éditions Blackie & Son (Glasgow and London), 1991, pp 116-178) Ils peuvent être notamment choisis parmi les alcools, les alpha-diols, les alkyl(C₁-C₂₀) phénols ou les acides gras polyéthoxylés, polypropoxylés ou polyglycérolés, ayant une chaîne grasse comportant par exemple de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et d'oxyde de propylène, les condensats d'oxyde d'éthylène et d'oxyde de propylène sur des alcools gras ; les amides gras polyéthoxylés ayant de préférence de 2 à 30 moles d'oxyde d'éthylène ; les amides gras polyglycérolés comportant en moyenne 1 à 5 groupements glycérol et en particulier 1,5 à 4 ; les esters d'acides gras du sorbitane éthoxylés ayant de 2 à 30 moles d'oxyde d'éthylène ; les esters d'acides gras du saccharose, les esters d'acide gras du polyéthylèneglycol, les alkyl(C₆-C₂₄)polyglucosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine; et leurs mélanges.

Les agents tensioactifs amphotères, utilisables dans les compositions selon la présente invention, peuvent être notamment des dérivés d'amines aliphatiques secondaires ou tertiaires, dans lesquels le groupe aliphatique est une chaîne linéaire ou ramifiée comportant 8 à 22 atomes de carbone et contenant, au moins un groupe anionique hydrosolubilisant tel que, par exemple, un groupe carboxylate, sulfonate, sulfate, phosphate ou phosphonate, on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL^{®}, tels que décrits dans les brevets US-2,528,378 et US-2,781,354 et classés dans le dictionnaire CTFA, 3^{ème} édition, 1982, sous les dénominations Amphocarboxyglycinate et Amphocarboxypropionate de structures respectives (1) et (2) :

R₂ -CONHCH₂CH₂ -N⁺(R₃)(R₄)(CH₂COO⁻) (1)

dans laquelle :
R₂ désigne représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ;
   Et

   R₂ -CONHCH₂CH₂ -N(B)(C) (2)

   dans laquelle :
   B représente -CH₂CH₂OX',
   C représente -(CH₂)_{z} -Y', avec z = 1 ou 2,
   X' représente le groupement -CH₂CH₂-COOH ou un atome d'hydrogène,
   Y' représente -COOH ou le groupe -CH₂ - CHOH - SO₃H,
   R₂ représente le groupe alkyle d'un acide R_{2'}-COOH présent dans l'huile de coprah ou dans l'huile de lin hydrolysée, un groupe alkyle, notamment en C₁₇ et sa forme iso, un groupe C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple on peut citer le cocoamphodiacétate commercialisé sous la dénomination commerciale MIRANOL^{®} C2M concentré par la société RHODIA.

Parmi les tensioactifs amphothères, on utilise de préférence les alkyl(C₈-C₂₀)bétaïnes telles que la cocobétaïne, les alkyl(C₈-C₂₀)amidoalkyl(C₆-C₈)bétaïnes telles que la cocamidobétaïne, les alkylamphodiacétates comme le cocoamphodiacétate disodique, et leurs mélanges.

La composition selon l'invention peut comprendre en outre un ou plusieurs tensioactifs cationiques bien connus en soi, tels que les sels d'amines grasses primaires, secondaires ou tertiaires, éventuellement polyoxyalkylénées, les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium, les dérivés d'imidazoline ; ou les oxydes d'amines à caractère cationique.

Les agents tensioactifs anioniques, non ioniques, amphotères et cationiques décrits ci-dessus peuvent être utilisés seuls ou en mélanges et leur quantité est inférieure à 4 %, plus préférentiellement comprise entre 0,01% et 4 % en poids, et mieux encore entre 0,1% et 2% en poids par rapport au poids total de la composition.

De préférence, la composition selon l'invention comprend un ou plusieurs polymères fixants et/ou une ou plusieurs silicones et/ou un ou plusieurs corps gras.

Les silicones utilisables en tant qu'additifs dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent être solubles ou insolubles dans la composition et en particulier être des polyorganosiloxanes insolubles dans la composition de l'invention. Elles peuvent se présenter sous forme d'huiles, de cires, de résines ou de gommes. Ces silicones sont différentes des alcoxysilanes à fonctions amines utilisées dans la présente invention.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les silicones cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE^{®} 7207 par UNION CARBIDE ou SILBIONE^{®} 70045 V2 par RHODIA, le décaméthylcyclopentasiloxane commercialisé sous le nom de VOLATILE SILICONE^{®} 7158 par UNION CARBIDE, et SILBIONE® 70045 V5 par RHODIA, ainsi que leurs mélanges.
   On peut également citer les cyclocopolymères du type diméthylsiloxanes/ méthylalkylsiloxane, tel que la SILICONE VOLATILE^{®} FZ 3109 commercialisée par la société UNION CARBIDE, de formule : 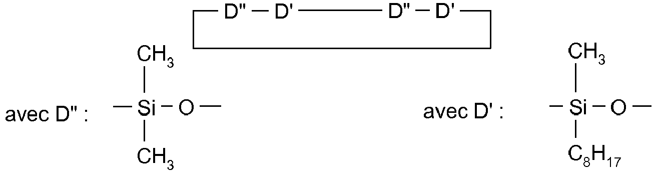
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des silicones non volatiles, et plus particulièrement des polyalkylsiloxanes, des polyarylsiloxanes, des polyalkylarylsiloxanes, des gommes et des résines de silicones, des polyorganosiloxanes modifiés par les groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polyalkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polyméthylsiloxanes à groupements terminaux diméthylsilanol connus sous le nom de dimethiconol (CTFA), tels que les huiles de la série 48 de la société RHODIA.

Dans cette classe de polyalkylsiloxanes, on peut également citer les produits commercialisés sous les dénominations "ABIL WAX® 9800 et 9801" par la société GOLDSCHMIDT qui sont des polyalkyl (C₁-C₂₀) siloxanes.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polyorganosiloxanes, ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

On peut citer plus particulièrement les produits suivants :
- les gommes de polydiméthylsiloxane,
- les gommes de polydiméthylsiloxane/méthylvinylsiloxane,
- les gommes de polydiméthylsiloxane/diphénylsiloxane,
- les gommes de polydiméthylsiloxane/phénylméthylsiloxane,
- les gommes de polydiméthylsiloxane /diphénylsiloxane/ méthylvinylsiloxane.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R3SiO_{1/2}, RSiO_{3/2} et SiO_{4/2}
dans lesquelles R représente un groupement hydrocarboné possédant 1 à 16 atomes de carbone ou un groupement phényle. Parmi ces produits, ceux particulièrement préférés sont ceux dans lesquels R désigne un groupe alkyle inférieur en C₁-C₄, plus particulièrement méthyle ou un groupe phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939, ou DOW CORNING 2-8299 par la société DOW CORNING ou le produit commercialisé sous la dénomination BELSIL ADM LOG 1 par la société WACKER. Les groupements aminés substitués sont en particulier des groupements aminoalkyles en C₁-C₄ ;
- des groupements thiols, comme les produits commercialisés sous les dénominations GP 72A et GP 71 de GENESEE.
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.
- des groupements hydroxylés, comme les polyorganoxiloxanes à fonction hydroxyalkyle ;
- des groupements alcoxyalkyle tels que, par exemple, les polyorganosiloxanes décrits dans le brevet US-A-4 957 732 ;
- des groupements anioniques du type carboxylique comme par exemple, dans le produits décrits dans le brevet EP 186 507 de la société CHISSO CORPORATION, ou de type alkyl-carboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations ABIL^{®} S201 ET ABIL^{®} S255 ;
- des groupements hydroxyacrylamino, comme les polyorganosiloxanes décrits dans la demande EP 342 834. On peut citer, par exemple, le produit Q2-8413 de la société DOW CORNING.

Les silicones telles que décrites ci-dessus peuvent être utilisées seules ou en mélange, en une quantité comprise entre 0,01 et 20% en poids, de préférence entre 0,1 et 5% en poids.

Les compositions de l'invention peuvent encore comporter des corps gras tels que les huiles minérales, végétales, animales et synthétiques, les cires, les esters gras, les alcools gras éthoxylés ou non éthoxylés, et les acides gras.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépin de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que Parléam^{®} ; les isoparaffines comme l'isohexadécane et l'isodécane.
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M ;

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les acides gras saturés ou insaturés sont choisis plus particulièrement parmi l'acide myristique, l'acide palmitique, l'acide stéarique, l'acide béhénique, l'acide oléïque, l'acide linoléïque, l'acide linolénique et l'acide isostéarique.

Les esters gras sont notamment les esters d'acides carboxyliques en particulier les esters mono, di, tri ou tétracarboxyliques.

Les esters d'acides carboxyliques sont notamment les esters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle ; le dioctanoate de propylène glycol et le diheptanoate de néopentyl glycol.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

Comme alcools gras on peut citer les alcools gras saturés ou insaturés, linéaires ou ramifiés ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

Les corps gras représentent en général de 0,1 à 50%; de préférence 1 à 30%, et encore plus préférentiellement de 2 à 20% en poids de la composition totale.

Comme indiqué précédemment les compositions peuvent comprendre des polymères fixants

On entend par polymère fixant tout polymère apte à conférer une forme à une chevelure ou à maintenir une chevelure dans une forme donnée.

Tous les polymères fixants anioniques, cationiques, amphotères, non ioniques et leurs mélanges utilisés dans la technique peuvent être utilisés dans les compositions selon la présente demande.

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 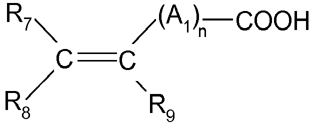 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les copolymères d'acide acrylique ou méthacrylique ou leurs sels et d'acrylamide vendus sous la forme de leurs sels de sodium sous les dénominations RETEN 421, 423 ou 425 par la Société HERCULES,
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois n^{os} 75370 et 75371 ou proposés sous la dénomination QUADRAMER par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF ;
   On peut aussi citer les copolymères acide méthacrylique/ acide acrylique/acrylate d'éthyle/méthacrylate de méthyle en dispersion aqueuse, commercialisé sous la dénomination AMERHOLD® DR 25 par la société AMERCHOL ;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ^{®} AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   - les fonctions anhydrides de ces copolymères étant éventuellement monoestérifiées ou monoamidifiées.

   Ces polymères sont par exemple décrits dans les brevets français n^{os} 2 350 384 et 2 357 241 de la demanderesse.
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan^{®} 500 et Flexan^{®} 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL.

Comme autre polymère fixant anionique utilisable selon l'invention, on peut citer le polymère anionique séquencé branché vendu sous la dénomination Fixate G-100 par la société NOVEON.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX^{®} A par la société BASF, le polymère vendu sous la dénomination Fixate G-100 par la société NOVEON.

Parmi les polymères fixants anioniques cités ci-dessus, on préfère plus particulièrement utiliser dans le cadre de la présente invention, les copolymères méthylvinyléther/anhydride maléique monoestérifiés vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF, les terpolymères vinylpyrrolidone/acide acrylique/méthacrylate de lauryle vendus sous la dénomination ACRYLIDONE^{®} LM par la société ISP, le polymère vendu sous la dénomination Fixate G-100 par la société NOVEON.

Les polymères filmogènes fixants cationiques utilisables selon la présente invention sont de préférence choisis parmi les polymères comportant des groupements amines primaires, secondaires, tertiaires et/ou quaternaires faisant partie de la chaîne polymère ou directement reliés à celle-ci, et ayant un poids moléculaire compris entre 500 et environ 5 000 000 et de préférence entre 1 000 et 3 000 000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 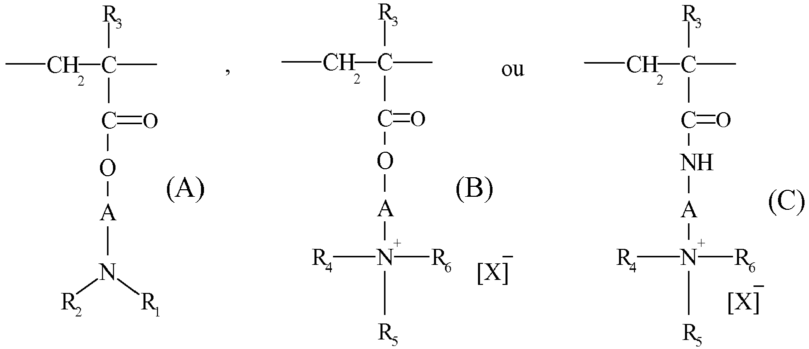 dans lesquelles:
   R3 désigne un atome d'hydrogène ou un radical CH3;
   A est un groupe alkyle linéaire ou ramifié comportant de 1 à 6 atomes de carbone ou un groupe hydroxyalkyle comportant de 1 à 4 atomes de carbone ;
   R4, R5, R6, identiques ou différents, représentent un groupe alkyle ayant de 1 à 18 atomes de carbone ou un radical benzyle;
   R1 et R2, identiques ou différents, représentent chacun un atome hydrogène ou un groupe alkyle ayant de 1 à 6 atomes de carbone;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones-acrylamides, acrylamides et méthacrylamides substitués sur l'azote par des groupes alkyles inférieurs (C1-C4), des groupes dérivés des acides acryliques ou méthacryliques ou leurs esters, des vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisé au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium tel que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT® " par la société ISP comme par exemple "GAFQUAT® 734" ou "GAFQUAT® 755" ou bien les produits dénommés "COPOLYMER® 845, 958 et 937". Ces polymères sont décrits en détail dans les brevets français 2.077.143 et 2.393.573,
   - les polymères à chaîne grasse et à motif vinylpyrrolidone, tels que les produits vendus sous la dénomination Stylèze W20 et Stylèze W10 par la société ISP,
   - les terpolymères méthacrylate de diméthylaminoéthyle/vinyl-caprolactame/vinylpyrrolidone tel que le produit commercialisé sous la dénomination GAFFIX VC 713 par la société ISP, et
   - et les copolymères vinylpyrrolidone/méthacrylamide de diméthylaminopropyle quaternisés tels que les produits commercialisés sous la dénomination "GAFQUAT® HS 100" par la société ISP.
(2) les gommes de guar cationiques, de préférence à ammonium quaternaire tels que ceux décrits dans les brevets américains 3.589.578 et 4.031.307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole ;
(4) les chitosanes ou leurs sels; les sels utilisables sont en particulier les acétate, lactate, glutamate, gluconate ou le pyrrolidone-carboxylate de chitosane.
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL .
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amines primaires ou secondaires.

Les polymères fixants amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les composés dont les groupes alkyles comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER^{®} ou LOVOCRYL^{®} 47 par la société NATIONAL STARCH.
(3) les polyaminoamides réticulés et acylés partiellement ou totalement dérivant de polyaminoamides de formule générale : 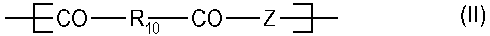 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 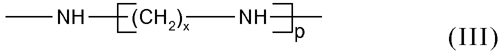 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (III) ci-dessus, dans lequel x=2 et p=1 et qui dérive de l'éthylène-diamine, ou le groupe dérivant de la pipérazine : 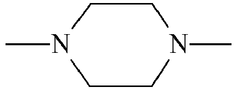
   c) dans les proportions de 0 à 20 % en moles, le groupe -NH-(CH₂)₆-NH- dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 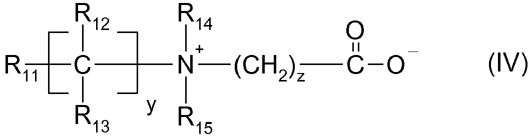 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle, tel que le produit vendu sous la dénomination DIAFORMER Z301 par la société SANDOZ.
(5) les polymères dérivés du chitosane comportant des motifs monomères répondant aux formules suivantes : 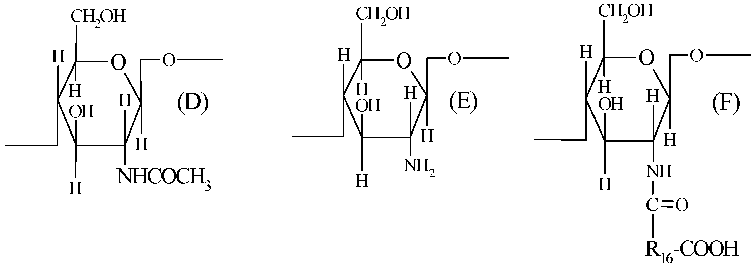 le motif (D) étant présent dans des proportions comprises entre 0 et 30%, le motif (E) dans des proportions comprises entre 5 et 50 % et le motif (F) dans des proportions comprises entre 30 et 90%, étant entendu que dans ce motif (F), R₁₆ représente un groupe de formule : 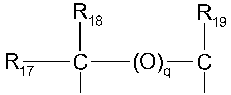 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 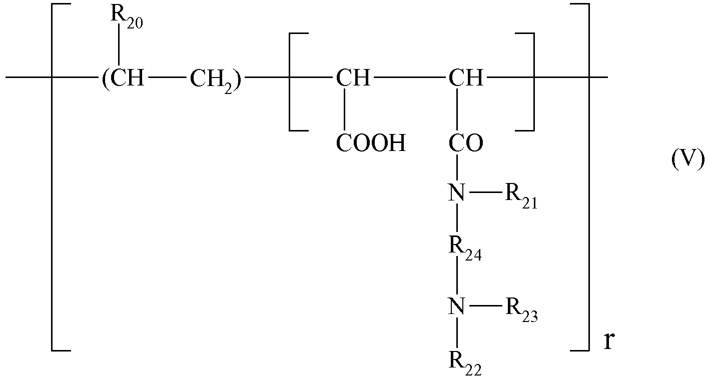 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂- , -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendus sous la dénomination « EVALSAN » par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 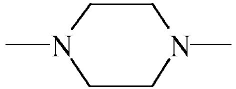 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')

      où D désigne un groupe 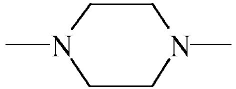 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyles et comportant un ou plusieurs atomes d'azote, l'atome d'azote étant substitué par une chaîne alkyle interrompue éventuellement par un atome d'oxygène et comportant obligatoirement une ou plusieurs fonctions carboxyles ou une ou plusieurs fonctions hydroxyles et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthyl-carboxyméthylammonio-éthylméthacrylate de méthyle vendu par exemple sous la dénomination DIAFORMER Z301 par la société SANDOZ.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame, tels que les homopolymères de vinylpyrrolidone, le polyvinylcaprolactame commercialisé sous la dénomination Luviskol^{®} PLUS par la société BASF ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.

Les groupes alkyles des polymères non ioniques mentionnés ci-dessus ont, de préférence, de 1 à 6 atomes de carbone.

Selon l'invention, on peut également utiliser des polymères fixants de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

Ces polymères peuvent être amphotères, anioniques ou non ioniques, et ils sont de préférence anioniques ou non ioniques.

De tels polymères sont, par exemple, les copolymères susceptibles d'être obtenus par polymérisation radicalaire à partir du mélange de monomères formé :
a) de 50 à 90 % en poids d'acrylate de tertiobutyle,
b) de 0 à 40 % en poids d'acide acrylique,
c) de 5 à 40 % en poids d'un macromère siliconé de formule 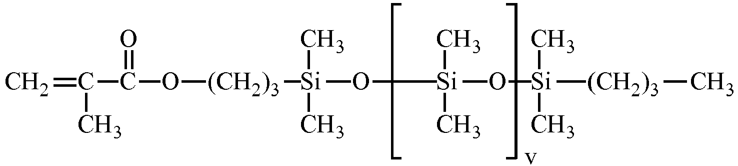
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex^{®} Silk commercialisé par la société BASF.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR^{®} et LUVISET^{®} Si PUR par la société BASF.

La concentration en polymère(s) fixant(s) utilisée dans les compositions selon la présente invention est comprise entre 0,1 et 20%, de préférence entre 0,5 et 10% en poids par rapport au poids total de la composition.

L'homme du métier saurait ajouter les additifs sans perturber les propriétés des compositions de l'invention.

Les compositions selon l'invention peuvent se présenter sous toutes les formes appropriées pour une application topique, notamment sous forme de solutions du type lotion ou sérum ; sous forme de gels aqueux ; sous forme d'émulsions obtenues par dispersion d'une phase grasse dans une phase aqueuse (H/E) ou inversement (E/H), de consistance liquide plus ou moins épaisse telle que des laits et des crèmes plus ou moins onctueuses ; sous forme de mousse, de cires, de pâtes, sous forme de spray, ou d'aérosol.

Lorsque la gomme de xanthane est utilisée à titre de gomme microbienne, la composition sera de préférence formulée en gel crème.

Lorsque la composition selon l'invention est conditionnée dans un dispositif aérosol, elle comprend un ou plusieurs agents propulseurs, qui peuvent être choisis parmi les hydrocarbures volatiles, tels que le N-butane, le propane, l'isobutane, le pentane, les hydrocarbures halogénés et leurs mélanges. On peut également utiliser en tant que propulseur le gaz carbonique, le protoxyde d'azote, le diméthyléther (DME), l'azote, ou l'air comprimé. On peut aussi utiliser des mélanges de propulseurs. De préférence, on utilise le diméthyl éther.

Avantageusement, le ou les agents propulseurs sont présents à une concentration comprise entre 5 et 90% en poids par rapport au poids total de la composition dans le dispositif aérosol, et, plus particulièrement, à une concentration comprise entre 10 et 60%.

La composition selon l'invention peut notamment être utilisée en application non rincée sur les cheveux.

Le ou les alcoxysilanes à fonction(s) basique(s) d'une part et la ou les gommes microbiennes d'autre part peuvent aussi être formulés séparément et mélangés juste avant l'application, ils peuvent être conditionnés dans un équipement à deux compartiments (bicompartimental), ils peuvent aussi être formulés séparément et appliqués l'un après l'autre sur les cheveux.

Les exemples qui suivent illustrent l'invention sans en limiter la portée.

### Exemples

Les compositions suivantes ont été réalisées :
Les concentrations sont exprimées en gramme de matières actives pour 100 grammes de composition.

### Exemples de formulations

### Exemple 1 : Gel-crème de coiffage :

| | |
|---|---|
| Aminopropyltriéthoxysilane (Dow Corning) | 10 % m.a. |
| HCl | qs pH=10.5 |
| Keltrol T (CP Kelco) | 2 % m.a. |
| Eau | qsp 100 % |

### Exemple 2 :Gel de coiffage en équipement bicompartimental :

### Compartiment A :

| | |
|---|---|
| Aminopropyltriéthoxysilane (Dow Corning) | 10 % m.a. |
| HCl | qs pH=10.5 |
| Jaguar P105 (Rhodia) | 1,5 % m.a. |
| Eau déminéralisée | qsp 100 % |

### Compartiment B :

| | |
|---|---|
| Keltrol T (CP Kelco) | 2 % m.a. |
| Eau | qsp 100 % |

Les contenus des 2 compartiments se mélangent à la sortie de l'équipement.

### Exemple 3 :Gel de coiffage en équipement bicompartimental :

### Compartiment A :

| | |
|---|---|
| N-(2-aminoéthyl)aminométhylphénéthyltriméthoxysilane (Gelest) | 10 % m.a. |
| Klucel G de Aqualon | |
| - Hercules (hydroxypropyl cellulose) | 2 % |
| Ethanol | 50 % |
| Eau déminéralisée | qsp 100 % |

### Compartiment B :

| | |
|---|---|
| Keltrol T (CP Kelco) | 2 % m.a. |
| Eau | qsp 100 % |

Les contenus des 2 compartiments se mélangent à la sortie de l'équipement.

### Application :

Les produits sont appliqués sur cheveux humides et laissés sécher librement, au brushing ou à l'aide de pinces plates.

### Résultats :

Sur cheveux abîmés (décolorés) : on obtient des effets coiffants et de soin (corps, souplesse, toucher doux et lisse) résistants à plusieurs shampooings.

Sur cheveux frisés, secs : on obtient des effets de contrôle de volume, de dessin de boucle et de soin (toucher doux et lisse) résistants à plusieurs shampooings.

## Revendications

1. Composition cosmétique non détergente comprenant un ou plusieurs alcoxysilane(s) présentant une ou plusieurs fonction(s) basique(s) et une ou plusieurs gomme(s) microbienne(s)

2. Composition selon la revendication 1 dans laquelle le ou les alcoxysilane(s) à fonction(s) basiques(s) sont choisis parmi les composés de formule (I) : 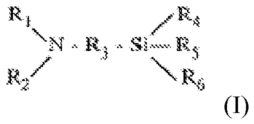 dans laquelle :
R₄ représente un halogène, un groupe OR' ou R'₁ ;
R₅ représente un halogène, un groupe OR" ou R'₂ ;
R₆ représente un halogène, un groupe OR"' ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires tels que des groupes acides ou amines, R₁, R₂, R', R" et R"' pouvant en outre désigner l'hydrogène, et deux au moins des groupes R₄, R₅ et R₆ étant différents des groupes R'₁, R'₂ et R'₃, les groupes R₁, R₂, R', R'₁, R'₂, R'₃, R"et R"' étant de préférence choisis parmi les radicaux alkyle de C₁-C₁₂, aryle de C₅ à C₁₄, alkyl(C₁-C₈)aryle de C₅ à C₁₄, et aryl(C₅-C₁₄)alkyle de C₁ à C₈ le groupe R₃ étant de préférence choisi parmi les radicaux alkylène en C₁-C₁₂, éventuellement substitué par un groupement amino, arylène en C₅-C₁₄, alkylène(C₁-C₈)arylène en C₅-C₁₄, et arylène(C₅-C₁₄)alkylène en C₁-C₈ ;
ou parmi les composés de formule (II) :
(R₂₁O)ₓ(R₂₂)_{y}Si-(B)ₚ-[NR₂₃-(B')_{p'}]_{q}-[NR'₂₃-(B")_{p"}]_{q'}-(R'₂₂)_{y'}(OR'₂₁)_{x'} (II)
dans laquelle :
R₂₁, R₂₂, R'₂₁ et R'₂₂ représentent chacun indépendamment une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x étant un entier variant de 1 à 3, y = 3-x, x' étant un entier variant de 1 à 3, y' = 3-x', p = 0 ou 1, p' = 0 ou 1, p" = 0 ou 1, q = 0 ou 1, q' = 0 ou 1, étant entendu que au moins q ou q' est différent de zéro,
B, B' et B" représentant chacun indépendamment un radical divalent alkylène linéaire ou ramifié en C₁-C₂₀,
R₂₃ et R'₂₃ représentent chacun indépendamment un atome d'hydrogène ou une chaîne hydrocarbonée saturée ou insaturée, linéaire ou ramifiée, contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₃-C₂₀, amine, amide, carboxyle, alcoxysilane, hydroxyle, carbonyle ou acyle,
avec de préférence, R₂₁ = R'_{21 ;} R₂₂ = R'_{22 ;} x = x' ; y = y' ; p = p' ; B = B' ; q = 1 et q' = 0 ;
ou parmi les composés de formule (III) : 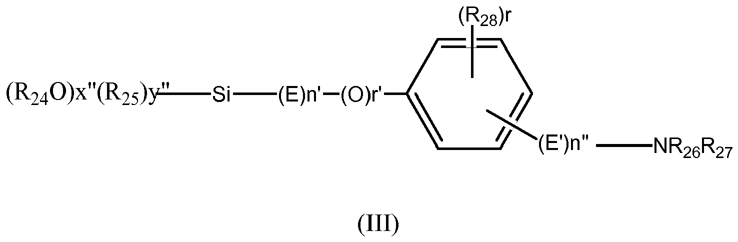 dans laquelle :
R₂₄ et R₂₅ représentent chacun indépendamment une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x" = 2 ou 3 ;
y" = 3-x" ;
n' = 0 ou 1 ;
n"= 0 ou 1 ;
E et E' représentent chacun indépendamment un radical divalent alkylène linéaire ou ramifié en C₁-C₂₀,
R₂₆ et R₂₇ représentent chacun indépendamment l'hydrogène ou une chaîne hydrocarbonée saturée ou insaturée, linéaire ou ramifiée, contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₁-C₂₀, amine, amide, carboxyle, alcoxysilane, hydroxyle, carbonyle ou acyle,
r est un entier variant de 0 à 4,
r' = 0 ou 1,
le ou les groupes R₂₈ représentent chacun indépendamment l'hydrogène ou une chaîne hydrocarbonée saturée ou insaturée, linéaire ou ramifiée, de préférence en C₁-C₁₀, contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₁-C₂₀, amine, amide, carboxyle, alcoxysilane, hydroxyle, carbonyle ou acyle ;
les chaînes hydrocarbonées comportant de préférence de 1 à 30 et mieux 1 à 10 atomes de carbone, le cycle aromatique comportant de préférence de 6 à 30 atomes de carbone.

3. Composition selon la revendication 1 ou 2 dans laquelle le ou les alcoxysilanes à fonction(s) basiques(s) sont choisis parmi les composés de formule (I) : 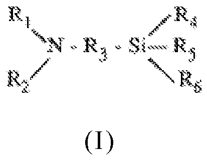 dans laquelle :
R₁ et R₂, indépendamment l'un de l'autre sont choisis parmi l'hydrogène et les groupes éthyle, propyle, aminoéthyle,
R₃ est choisi parmi les groupes éthyle, propyle et méthylphénéthyle,
R₄, R₅ et R₆ indépendamment l'un de l'autre sont choisis parmi les groupes méthyle, méthoxy, éthoxy.

4. Composition cosmétique selon l'une quelconque des revendications 1 à 3, **caractérisée en ce que** l'alcoxysilane à fonction(s) basique(s) est choisi parmi les composés suivants : le 3-aminopropyltriéthoxysilane, le 3-aminoéthyltriéthoxysilane, le 3-aminopropylméthyldiéthoxysilane, le N-(2-aminoéthyl)-3-aminopropyltriéthoxysilane et le N-(2-aminoéthylaminométhyl)phénéthyltriméthoxysilane.

5. Composition cosmétique selon l'une quelconque des revendications 1 à 4, **caractérisée en ce** le ou les alcoxysilane(s) à fonction(s) basique(s) sont utilisés en une quantité allant de 0,1 à 20 %, de préférence entre 1 et 15 % en poids du poids total de la composition.

6. Composition cosmétique selon l'une quelconque des revendications précédentes **caractérisée par le fait que** la gomme microbienne est choisie parmi les gommes de scléroglucane, les gommes de gellane, les gommes de pullulane, les gommes de Curdlar, les gommes de xanthane, les gommes de grifolane, les gommes de lentinane, les gommes de Schizophyllane, les gommes de spirulinane et les gommes de krestine.

7. Composition cosmétique selon la revendication précédente **caractérisée par le fait que** la gomme microbienne est choisie parmi les gommes de scléroglucane et de xanthane.

8. Composition cosmétique selon l'une quelconque des revendications précédentes, **caractérisée en ce que** la ou les gommes microbiennes sont présentes dans la composition en une quantité allant de 0,01 à 10 % en poids du poids total de la composition, de préférence entre 0,1 et 4 %.

9. Composition cosmétique selon l'une quelconque des revendications précédentes, **caractérisée en ce que** le rapport pondéral entre la ou les gomme(s) microbienne(s) et le ou les alcoxysilanes à fonctions amines va de 1/20 à 1/3.

10. Composition cosmétique selon l'une quelconque des revendications précédentes **caractérisée en ce que** le milieu est aqueux ou hydroalcoolique.

11. Composition cosmétique selon l'une quelconque des revendications précédentes, **caractérisée en ce qu'**elle comprend un ou plusieurs agents épaississants additionnels différents des gommes microbiennes choisis de préférence parmi les polymères et copolymères carboxyvinyliques, les polymères et copolymères (alkyl)acryliques, les polymères et copolymères (alkyl)acrylamides, les poly(oxyalkylène)glycols, les esters de poly(oxyalkylène)glycols, les dérivés de cellulose, les alginates, les dérivés d'amidon, les gommes naturelles telles que les gommes de guar ou de caroube, les dérivés de chitine et de chitosane, les carraghénanes, les argiles, et leur mélanges.

12. Composition cosmétique selon l'une quelconque des revendications précédentes, **caractérisée en ce qu'**elle comprend un ou plusieurs tensioactifs choisis parmi les tensioactifs anioniques, non ioniques, amphotères ou cationiques, ou des mélanges de ceux-ci.

13. Composition cosmétique selon l'une quelconque des revendications précédentes **caractérisée en ce qu'**elle comprend en outre un ou plusieurs polymères fixants et/ou une ou plusieurs silicones et/ou un ou plusieurs corps gras.

14. Utilisation pour le traitement des matières kératiniques, notamment des cheveux, et en particulier des cheveux abîmés, frisés, secs ou fins de la composition selon l'une quelconque des revendications 1 à 13.

15. Procédé de mise en forme des cheveux comprenant l'application sur les cheveux de la composition selon l'une quelconque des revendications 1 à 13.

## Patentansprüche

1. Nicht waschaktive kosmetische Zusammensetzung, umfassend ein oder mehrere Alkoxysilane mit einer oder mehreren basischen Funktionen und einem oder mehreren mikrobiellen Gummen.

2. Zusammensetzung nach Anspruch 1, wobei das Alkoxysilan bzw. die Alkoxysilane mit einer oder mehreren basischen Funktionen ausgewählt sind aus Verbindungen der Formel (I): 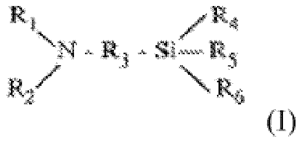 worin:
R₄ für ein Halogen oder eine Gruppe OR' oder R'₁ steht;
R₅ für ein Halogen oder eine Gruppe OR'' oder R'₂ steht;
R₆ für ein Halogen oder eine Gruppe OR''' oder R'₃ steht;
R₁, R₂, R₃, R', R'', R"', R'₁, R'₂ und R'₃ unabhängig voneinander für eine lineare oder verzweigte, gesättigte oder ungesättigte Kohlenwasserstoffgruppe, die gegebenenfalls zusätzliche chemische Gruppen wie Säure- oder Amingruppen trägt, stehen, wobei R₁, R₂, R', R'' und R''' außerdem Wasserstoff bedeuten können und mindestens zwei der Gruppen R₄, R₅ und R₆ von den Gruppen R'₁, R'₂ und R'₃ verschieden sind, wobei die Gruppen R₁, R₂, R', R'₁, R'₂, R'₃, R'' und R"' vorzugsweise aus C₁-C₁₂-Alkyl-, C₅-C₁₄-Aryl-, C₁-C₈-Alkyl-C₅-C₁₄-aryl- und C₅-C₁₄-Aryl-C₁-C₈-alkylresten ausgewählt sind, wobei die Gruppe R₃ vorzugsweise aus gegebenenfalls durch eine Aminogruppe substituierten C₁-C₁₂-Alkylen-, C₅-C₁₄-Arylen-, C₁-C₈-Alkylen-C₅-C₁₄-arylen- und C₅-C₁₄-Arylen-C₁-C₈-alkylenresten ausgewählt sind;
oder aus den Verbindungen der Formel (II):
(R₂₁O)ₓ(R₂₂)_{y}Si-(B)ₚ-[NR₂₃-(B')_{p'}]_{q}-[NR'₂₃-(B'')_{p''}]_{q'}-(R'₂₂)_{y'} (OR'₂₁)_{x'} (II)
worin:
R₂₁, R₂₂, R'₂₁ und R'₂₂ jeweils unabhängig für eine lineare oder verzweigte, gesättigte oder ungesättigte Kohlenwasserstoffkette, die gegebenenfalls ein oder mehrere Heteroatome enthält und gegebenenfalls durch eine oder mehrere aus Ether-, Ester-, Amin-, Amid-, Carboxyl-, Hydroxyl- und Carbonylgruppen ausgewählte Gruppen unterbrochen oder substituiert ist, stehen,
wobei x für eine ganze Zahl im Bereich von 1 bis 3 steht, y = 3-x, x' für eine ganze Zahl von 1 bis 3 steht, y' = 3-x', p = 0 oder 1, p' = 0 oder 1, p'' = 0 oder 1, q = 0 oder 1, q' = 0 oder 1, mit der Maßgabe, dass mindestens q oder q' von null verschieden ist,
B, B' und B'' jeweils unabhängig für einen linearen oder verzweigten zweiwertigen C₁-C₂₀-Alkylenrest stehen,
R₂₃ und R'₂₃ jeweils unabhängig für ein Wasserstoffatom oder eine lineare oder verzweigte, gesättigte oder ungesättigte Kohlenwasserstoffkette, die gegebenenfalls ein oder mehrere Heteroatome enthält und gegebenenfalls durch eine oder mehrere aus Ether-, C₁-C₂₀-Alkoholester-, Amin-, Carboxyl-, Alkoxysilan-, C₆-C₃₀-Aryl-, Hydroxyl-oder Carbonylgruppen ausgewählte Gruppen unterbrochen oder substituiert ist, oder einen heterocyclischen oder nicht heterocyclischen aromatischen Ring, der gegebenenfalls durch ein oder mehrere C₃-C₂₀-Alkoholester-, Amin-, Amid-, Carboxyl-, Alkoxysilan-, Hydroxyl-, Carbonyl- oder Acylgruppen substituiert ist, stehen,
wobei vorzugsweise R₂₁ = R'₂₁; R₂₂ = R'₂₂; x = x' ; y = y' ; p = p' ; B = B' ; q = 1 und q' = 0;
oder aus den Verbindungen der Formel (III): 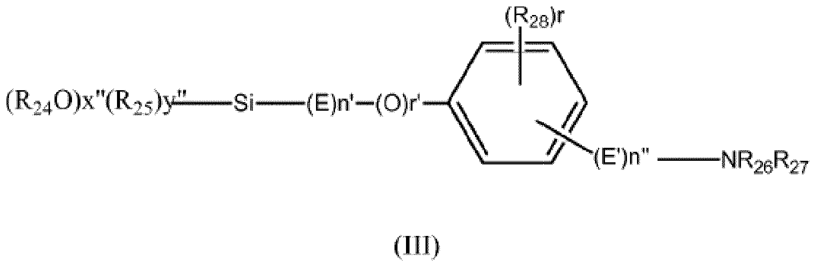 worin:
R₂₄ und R₂₅ jeweils unabhängig für eine lineare oder verzweigte, gesättigte oder ungesättigte Kohlenwasserstoffkette, die gegebenenfalls ein oder mehrere Heteroatome enthält und gegebenenfalls durch eine oder mehrere aus Ether-, Ester-, Amin-, Amid-, Carboxyl-, Hydroxyl- und Carbonylgruppen ausgewählte Gruppen unterbrochen oder substituiert ist, stehen,
x" - 2 oder 3;
y'' = 3-x'';
n' = 0 oder 1;
n''= 0 oder 1;
E und E' jeweils unabhängig für einen linearen oder verzweigten zweiwertigen C₁-C₂₀-Alkylenrest stehen,
R₂₆ und R₂₇ jeweils unabhängig für Wasserstoff oder eine lineare oder verzweigte, gesättigte oder ungesättigte Kohlenwasserstoffkette, die gegebenenfalls ein oder mehrere Heteroatome enthält und gegebenenfalls durch eine oder mehrere aus Ether-, C₁-C₂₀-Alkoholester-, Amin-, Carboxyl-, Alkoxysilan-, C₆-C₃₀-Aryl-, Hydroxyl- oder Carbonylgruppen ausgewählte Gruppen unterbrochen oder substituiert ist, oder einen heterocyclischen oder nicht heterocyclischen aromatischen Ring, der gegebenenfalls durch ein oder mehrere C₁-C₂₀-Alkoholester-, Amin-, Amid-, Carboxyl-, Alkoxysilan-, Hydroxyl-, Carbonyl- oder Acylgruppen substituiert ist, stehen,
r für eine ganze Zahl im Bereich von 0 bis 4 steht,
r' = 0 oder 1,
die Gruppe bzw. Gruppen R₂₈ jeweils unabhängig für Wasserstoff oder eine lineare oder verzweigte, gesättigte oder ungesättigte Kohlenwasserstoffkette, vorzugsweise C₁-C₁₀-Kohlenwasserstoffkette, die gegebenenfalls ein oder mehrere Heteroatome enthält und gegebenenfalls durch eine oder mehrere aus Ether-, C₁-C₂₀-Alkoholester-, Amin-, Carboxyl-, Alkoxysilan-, C₆-C₃₀-Aryl-, Hydroxyl- oder Carbonylgruppen ausgewählte Gruppen unterbrochen oder substituiert ist, oder einen heterocyclischen oder nicht heterocyclischen aromatischen Ring, der gegebenenfalls durch ein oder mehrere C₁-C₂₀-Alkoholester-, Amin-, Amid-, Carboxyl-, Alkoxysilan-, Hydroxyl-, Carbonyl- oder Acylgruppen substituiert ist, stehen;
wobei die Kohlenwasserstoffketten vorzugsweise 1 bis 30 und noch besser 1 bis 10 Kohlenstoffatome enthalten und der aromatische Ring vorzugsweise 6 bis 30 Kohlenstoffatome enthält.

3. Zusammensetzung nach Anspruch 1 oder 2, wobei das Alkoxysilan bzw. die Alkoxysilane mit einer oder mehreren basischen Funktionen ausgewählt sind aus Verbindungen der Formel (I): 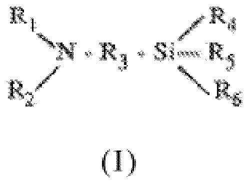 worin:
R₁ und R₂ unabhängig voneinander aus Wasserstoff und Ethyl-, Propyl- und Aminoethylgruppen ausgewählt sind,
R₃ aus Ethyl-, Propyl- und Methylphenethylgruppen ausgewählt ist,
R₄, R₅ und R₆ unabhängig voneinander aus Methyl-, Methoxy- und Ethoxygruppen ausgewählt sind.

4. Kosmetische Zusammensetzung nach einem der Ansprüche 1 bis 3, **dadurch gekennzeichnet, dass** das Alkoxysilan mit einer oder mehreren basischen Funktionen aus den folgenden Verbindungen ausgewählt ist: 3-Aminopropyltriethoxysilan, 3-Aminoethyltriethoxysilan, 3-Aminopropylmethyldiethoxysilan, N-(2-Aminoethyl)-3-aminopropyl-triethoxysilan und N-(2-Aminoethylaminomethyl)-phenethyltrimethoxysilan.

5. Kosmetische Zusammensetzung nach einem der Ansprüche 1 bis 4, **dadurch gekennzeichnet, dass** das Alkoxysilan bzw. die Alkoxysilane mit einer oder mehreren basischen Funktionen in einer Menge im Bereich von 0,1 bis 20 Gew.-%, vorzugsweise zwischen 1 und 15 Gew.-%, des Gesamtgewichts der Zusammensetzung verwendet werden.

6. Kosmetische Zusammensetzung nach einem der vorhergehenden Ansprüche, **dadurch gekennzeichnet, dass** das mikrobielle Gummi aus Skleroglucan-Gummen, Gellan-Gummen, Pullulan-Gummen, Curdlar-Gummen, Xanthan-Gummen, Grifolan-Gummen, Lentinan-Gummen, Schizophyllan-Gummen, Spirulinan-Gummen und Krestin-Gummen ausgewählt ist.

7. Kosmetische Zusammensetzung nach dem vorhergehenden Anspruch, **dadurch gekennzeichnet, dass** das mikrobielle Gummi aus Skleroglucan- und Xanthan-Gummen ausgewählt ist.

8. Kosmetische Zusammensetzung nach einem der vorhergehenden Ansprüche, **dadurch gekennzeichnet, dass** das mikrobielle Gummi bzw. die mikrobiellen Gummen in einer Menge im Bereich von 0,01 bis 10 Gew.-% des Gesamtgewichts der Zusammensetzung, vorzugsweise zwischen 0,1 und 4 Gew.-%, vorliegen.

9. Kosmetische Zusammensetzung nach einem der vorhergehenden Ansprüche, **dadurch gekennzeichnet, dass** das Gewichtsverhältnis zwischen dem mikrobiellen Gummi bzw. den mikrobiellen Gummen und dem Alkoxysilan bzw. den Alkoxysilanen mit Aminfunktionen 1/20 bis 1/3 beträgt.

10. Kosmetische Zusammensetzung nach einem der vorhergehenden Ansprüche, **dadurch gekennzeichnet, dass** das Medium wässrig oder wässrig-alkoholisch ist.

11. Kosmetische Zusammensetzung nach einem der vorhergehenden Ansprüche, **dadurch gekennzeichnet, dass** sie ein oder mehrere zusätzliche, von den mikrobiellen Gummen verschiedene Verdickungsmittel umfasst, die vorzugsweise aus Carboxyvinylpolymeren und -copolymeren, (Alkyl)acrylpolymeren und -copolymeren, (Alkyl)acrylamidpolymeren und - copolymeren, Poly(oxyalkylen)glykolen, Poly(oxy-alkylen)glykolestern, Cellulosederivaten, Alginaten, Stärkederivaten, natürlichen Gummen wie Guar-Gummi oder Johannisbrotkernmehl, Chitin- und Chitosanderivaten, Carrageenanen, Tonen und Mischungen davon ausgewählt sind.

12. Kosmetische Zusammensetzung nach einem der vorhergehenden Ansprüche, **dadurch gekennzeichnet, dass** sie ein oder mehrere Tenside, die aus anionischen, nichtionischen, amphoteren oder kationischen Tensiden oder Mischungen davon ausgewählt sind, umfasst.

13. Kosmetische Zusammensetzung nach einem der vorhergehenden Ansprüche, **dadurch gekennzeichnet, dass** sie außerdem ein oder mehrere festigende Polymere und/oder ein oder mehrere Silikone und/oder eine oder mehrere Fettsubstanzen umfasst.

14. Verwendung der Zusammensetzung nach einem der Ansprüche 1 bis 13 zur Behandlung von Keratinmaterialien, insbesondere Haaren und speziell geschädigten, lockigen, trockenen oder feinen Haaren.

15. Verfahren zur Gestaltung von Haaren, bei dem man auf die Haare die Zusammensetzung nach einem der Ansprüche 1 bis 13 aufbringt.

## Claims

1. Nondetergent cosmetic composition comprising one or more alkoxysilane(s) having one or more basic function(s) and one or more microbial gum (s) .

2. Composition according to Claim 1, in which the alkoxysilane(s) comprising a basic function or basic functions is (are) chosen from the compounds of formula (I): 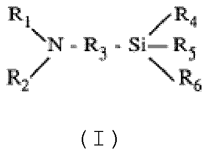 in which:
R₄ represents a halogen, a group OR' or R'₁;
R₅ represents a halogen, a group OR'' or R'₂;
R₆ represents a halogen, a group OR''' or R'₃;
R₁, R₂, R₃, R', R'', R''', R'₁, R'₂, R'₃ represent, independently of one another, a linear or branched, saturated or unsaturated hydrocarbon-based group optionally bearing additional chemical groups such as acid or amine groups, it also being possible for R₁, R₂, R', R'' and R"' to denote hydrogen, and at least two of the R₄, R₅ and R₆ groups being different from the R'₁, R'₂ and R'₃ groups, the R₁, R₂, R', R'₁, R'₂, R'₃, R'' and R''' groups preferably being chosen from C₁-C₁₂ alkyl, C₅-C₁₄ aryl, (C₁-C₈) alkyl (C₅-C₁₄) aryl and (C₅-C₁₄) aryl (C₁-C₈) alkyl radicals, the R₃ group preferably being chosen from the radicals: C₁-C₁₂ alkylene, optionally substituted with an amino group, C₅-C₁₄ arylene, (C₁-C₈) alkylene (C₅-C₁₄) arylene and (C₅-C₁₄) arylene (C₁-C₈) alkylene;
or from the compounds of formula (II):
(R₂₁O) ₓ (R₂₂) ySi- (B) ₚ- [NR₂₃- (B') _{p'} ]_{q}- [NR'₂₃-(B") _{p''}]_{q'}-(R'₂₂)_{y'} (OR'₂₁)_{x'} (II)
in which:
R₂₁, R₂₂, R'₂₁ and R'₂₂ each independently represent a linear or branched, saturated or unsaturated hydrocarbon-based chain optionally containing one or more heteroatoms, optionally interrupted or substituted with one or more groups chosen from ether, ester, amine, amide, carboxyl, hydroxyl and carbonyl groups.
x being an integer ranging from 1 to 3, y = 3-x, x' being an integer ranging from 1 to 3, y' = 3-x', p = 0 or 1, p' = 0 or 1, p'' = 0 or 1, q = 0 or 1, q' = 0 or 1, it being understood that at least q or q' is other than zero,
B, B' and B'' each independently represent a linear or branched C₁-C₂₀ alkylene divalent radical,
R₂₃ and R'₂₃ each independently represent a hydrogen atom or a linear or branched, saturated or unsaturated hydrocarbon-based chain optionally containing one or more heteroatoms, optionally interrupted or substituted with one or more ether, C₁-C₂₀ alkohol ester, amine, carboxyl, alkoxysilane, C₆-C₃₀ aryl, hydroxyl or carbonyl groups, or a heterocyclic or nonheterocyclic aromatic ring optionally substituted with one or more C₃-C₂₀ alkohol ester, amine, amide, carboxyl, alkoxysilane, hydroxyl, carbonyl or acyl groups,
with preference, R₂₁ = R'₂₁; R₂₂ = R'_{22;} x = x'; y = y'; p = p'; B = B'; q = 1 and q' = 0;
or from the compounds of the formula (III): 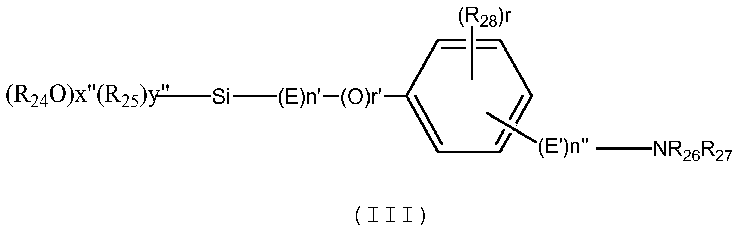 in which:
R₂₄ and R₂₅ each independently represent a linear or branched, saturated or unsaturated hydrocarbon-based chain optionally containing one or more heteroatoms, optionally interrupted or substituted with one or more groups chosen from ether, ester, amine, amide, carboxyl, hydroxyl and carbonyl groups,
x'' = 2 or 3;
y'' = 3-x'';
n ' = 0 or 1;
n''= 0 or 1;
E and E' each independently represent a linear or branched C₁-C₂₀ alkylene divalent radical,
R₂₆ and R₂₇ each independently represent hydrogen or a linear or branched, saturated or unsaturated hydrocarbon-based chain optionally containing one or more heteroatoms, optionally interrupted or substituted with one or more ether, C₁-C₂₀ alkohol ester, amine, carboxyl, alkoxysilane, C₆-C₃₀ aryl, hydroxyl or carbonyl groups, or a heterocyclic or nonheterocyclic aromatic ring optionally substituted with one or more C₁-C₂₀ alkohol ester, amine, amide, carboxyl, alkoxysilane, hydroxyl, carbonyl or acyl groups,
r is an integer ranging from 0 to 4,
r' = 0 or 1,
the R₂₈ group(s) each independently repesent(s) hydrogen or a linear or branched, saturated or unsaturated, preferably C₁-C₁₀, hydrocarbon-based chain optionally containing one or more heteroatoms, optionally interrupted or substituted with one or more ether, C₁-C₂₀ alkohol ester, amine, carboxyl, alkoxysilane, C₆-C₃₀ aryl, hydroxyl or carbonyl groups, or a heterocyclic or non-heterocyclic aromatic ring optionally substituted with one or more C₁-C₂₀ alkohol ester, amine, amide, carboxyl, alkoxysilane, hydroxyl, carbonyl or acyl groups;
the hydrocarbon-based chains preferably containing from 1 to 30, and better still 1 to 10, carbon atoms, the aromatic ring preferably containing from 6 to 30 carbon atoms.

3. Composition according to Claim 1 or 2, in which the alkoxysilane(s) comprising a basic function or basic functions is (are) chosen from the compounds of formula (I): 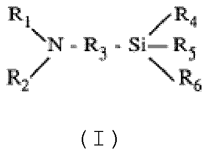 in which:
R₁ and R₂, independently of one another, are chosen from hydrogen and ethyl, propyl and aminoethyl groups,
R₃ is chosen from ethyl, propyl and methylphenethyl groups,
R₄, R₅ and R₆, independently of one another, are chosen from methyl, methoxy and ethoxy groups.

4. Cosmetic composition according to any one of Claims 1 to 3, **characterized in that** the alkoxysilane comprising a basic function or basic functions is chosen from the following compounds: 3-aminopropyltriethoxysilane, 3-aminoethyltriethoxysilane, 3-aminopropylmethyldiethoxysilane, N-(2-aminoethyl)-3-aminopropyltriethoxysilane and N-(2-aminoethylaminomethyl)phenethyltrimethoxysilane.

5. Cosmetic composition according to any one of Claims 1 to 4, **characterized in that** the alkoxysilane(s) comprising a basic function or basic functions is (are) used in an amount ranging from 0.1% to 20%, preferably between 1% and 15 % by weight of the total weight of the composition.

6. Cosmetic composition according to any one of the preceding claims, **characterized in that** the microbial gum is chosen from scleroglucan gums, gellan gums, pullulan gums, curdlan gums, xanthan gums, grifolan gums, lentinan gums, schizophyllan gums, spirulinan gums and krestin gums.

7. Cosmetic composition according to the preceding claim, **characterized in that** the microbial gum is chosen from scleroglucan gums and xanthan gums.

8. Cosmetic composition according to any one of the preceding claims, **characterized in that** the microbial gum(s) is (are) present in the composition in an amount ranging from 0.01% to 10% by weight of the total weight of the composition, preferably between 0.1% and 4%.

9. Cosmetic composition according to any one of the preceding claims, **characterized in that** the weight ratio between the microbial gum(s) and the alkoxysilane(s) comprising amine functions ranges from 1/20 to 1/3.

10. Cosmetic composition according to any one of the preceding claims, **characterized in that** the medium is aqueous or aqueous-alcoholic.

11. Cosmetic composition according to any one of the preceding claims, **characterized in that** it comprises one or more additional thickeners different from the microbial gums, preferably chosen from carboxyvinyl polymers and copolymers, (alkyl)acrylic polymers and copolymers, (alkyl)acrylamide polymers and copolymers, poly(oxyalkylene) glycols, poly(oxyalkylene) glycol esters, cellulose derivatives, alginates, starch derivatives, natural gums such as guar or carob gums, derivatives of chitin and of chitosan, carrageenans, clays, and mixtures thereof.

12. Cosmetic composition according to any one of the preceding claims, **characterized in that** it comprises one or more surfactants chosen from anionic, nonionic, amphoteric or cationic surfactants, or mixtures thereof.

13. Cosmetic composition according to any one of the preceding claims, **characterized in that** it also comprises one or more fixing polymers and/or one or more silicones and/or one or more fatty substances.

14. Use, for the treatment of keratin materials, especially the hair, and in particular damaged, curly, dry or fine hair, of the composition according to any one of Claims 1 to 13.

15. Method of hair shaping comprising the application of the composition according to any one of Claims 1 to 13 to the hair.
